# EUROPÄISCHE PATENTANMELDUNG

(11) **EP 2 174 926 A2**
(43) Veröffentlichungstag der Anmeldung: **14.04.2010**
(21) Anmeldenummer: 09012242.5
(22) Anmeldetag: 26.09.2009
(51) Int. Cl.: C07C 68/02, C07C 69/96

(54) **Verfahren zur Herstellung von Diarylcarbonaten**

(30) Priorität: 08.10.2008 DE 102008050828
(71) Anmelder: Bayer MaterialScience AG, 51368 Leverkusen (DE)
(72) Erfinder: Ooms, Pieter, Dr., 47800 Krefeld (DE)

(57) **Zusammenfassung**

Die Erfindung betrifft ein Verfahren zur Herstellung von Diarylcarbonaten durch Umsetzung von Monophenolen mit Phosgen oder Chlorkohlensäurearylestern unter Abspaltung von Chlorwasserstoff in Gegenwart von Mischhydroxiden von Elementen aus den Gruppen 2-14 des periodischen Systems (IUPAC, neu) als heterogene Katalysatoren.

## Beschreibung

Die Erfindung betrifft ein Verfahren zur Herstellung von Diarylcarbonaten durch Umsetzung von aromatischen Monohydroxyverbindungen mit Phosgen oder Chlorkohlensäurearylestern unter Abspaltung von Chlorwasserstoff in Gegenwart von Mischhydroxiden von Elementen aus den Gruppen 2 - 14 des periodischen Systems (IUPAC, neu) als heterogene Katalysatoren.

Diarylcarbonate eignen sich zur Herstellung von Polycarbonaten nach dem Schmelzeumesterungsverfahren (siehe z.B. in Chemistry and Physics of Polycarbonates, Polymer Reviews, H. Schnell, Vol. 9, John Wiley and Sons, Inc. (1964)), zur Herstellung von Phenylurethanen oder sind Vorprodukte von Wirkstoffen aus dem Pharma- und Pflanzenschutzsektor.

Es ist bekannt, dass Diarylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann. In jedem Falle werden große Mengen Kochsalz als Nebenprodukt erhalten. Ferner muss Sorge für die Lösungsmittel-Rückgewinnung getragen werden.

Man hat daher eine Kondensation ohne Mitverwendung von Lösungsmitteln und Natronlauge in Gegenwart von Tetramethylammoniumhalogeniden als Katalysatoren vorgeschlagen (US-A-2 837 555). Hier sind aber die benötigten Katalysatormengen relativ groß. Man muss in der Regel mit 5 bis 7 Gew.-% Katalysator, bezogen auf die eingesetzte Phenolmenge, arbeiten, um wirtschaftliche Reaktionsgeschwindigkeiten zu erhalten; die Reaktionstemperaturen von 180°C bis 215°C bringen zusätzlich die Gefahr einer Zersetzung der thermolabilen Tetramethylammoniumhalogenide mit sich. Der Katalysator muss ferner anschließend durch Waschen mit Wasser entfernt werden, wodurch seine Rückgewinnung erheblich erschwert wird. Darüber hinaus wird weit mehr als die stöchiometrisch notwendige Menge an Phosgen verbraucht.

Nach einem weiteren Verfahren (US-A-3 234 263) werden Diarylcarbonate durch Erhitzen von Chlorkohlensäurearylestern in Gegenwart großer Mengen (Erd)alkaliverbindungen mit tertiären Stickstoffbasen als Katalysatoren erhalten. Dieses Verfahren hat jedoch den Nachteil, dass man hohe Temperaturen anwenden und die Katalysatoren wie (Erd)Alkaliverbindungen teilweise lösen muss, um auch nur annähernd auf wirtschaftlich vertretbare Reaktionszeiten zu kommen. Bei diesem Verfahren geht die Hälfte des ursprünglich eingesetzten Phosgens in Form von CO₂ verloren. Außerdem muss man in einem vorgelagerten separaten Verfahrensschritt die Chlorkohlensäureester synthetisieren.

Nach der US-A-2 362 865 erhält man Diarylcarbonate durch Phosgenierung von Monophenolen in Gegenwart von metallischem Titan, Eisen, Zink und Zinn oder in Form ihrer löslichen Salze, besonders der Chloride und Phenolate. Obwohl sehr gute Ausbeuten erhalten werden, ist es schwierig, die Katalysatoren von den Produkten zu trennen. Man muss sogar bei Destillationen mit einer gewissen Flüchtigkeit dieser Verbindungen und auch mit thermischen Zersetzungen durch diese Verbindungen rechnen, die zu Verunreinigungen, Qualitätsminderungen und Ausbeuteeinbußen führen.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird nach der Lehre der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teuer. Weiter werden in WO 91/06526 Metallsalze auf porösen Trägern als Katalysatoren für die erfindungsgemäßen Umsetzungen beschrieben. Wie aus den Versuchsbeispielen hervorgeht, ist eine vollkontinuierliche Phosgenierung von Phenol an solchen Katalysatoren nur in der Gasphase möglich, was aber relativ hohe Reaktionstemperaturen und die Gefahr der Zersetzung des empfindlichen Chlorameisensäurephenylesters mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Die Aufgabe der Erfindung bestand also darin, einfach zugängliche, wirksame heterogene Katalysatoren zu entwickeln.

Es wurde nun gefunden, dass Mischhydroxide von Elementen aus den Gruppen 2-14 des periodischen Systems (IUPAC, neu) wie beispielsweise Hydrotalcit geeignete Katalysatoren für die Umsetzung von Phosgen oder Chlorkohlensäurearylestern mit Monophenolen zu Diarylcarbonaten darstellen, wobei das gebildete Chlorwasserstoff als Edukt in anderen Prozesse oder durch Oxidation zu Chlor wiederverwendet werden kann .

Das erfindungsgemäße Verfahren hat den großen Vorteil, sehr hohe Selektivitäten und gute Phenolumsätze zu erreichen um so ein Produkt mit hoher Reinheit zu erlangen. Darüber hinaus kann man den Katalysator sehr leicht abtrennen und somit die Aufarbeitung wesentlich vereinfachen.

Der Gegenstand der vorliegenden Erfindung ist demnach ein Verfahren zur Herstellung von Diarylcarbonaten durch Umsetzung von Monophenolen mit Phosgen oder Chlorameisensäurearylestern, das dadurch gekennzeichnet ist, dass man in Gegenwart von Mischhydroxiden von Elementen aus den Gruppen 2-14 des periodischen Systems (IUPAC, neu) als heterogene Katalysatoren arbeitet.

Monophenole für das erfindungsgemäße Verfahren sind solche der Formel

Ar-OH (I),

worin
- Ar: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C₁-C₄-Alkyl, geradkettiges oder verzweigtes C₁-C₄-Alkoxy, geradkettiges oder verzweigtes C₁-C₄-Alkoxycarbonyl, die mit Phenyl, Cyano und Halogen (z.B. F, Cl, Br) substituiert sein können und wobei weiterhin die heterocyclischen Reste mit einem ankondensierten Benzolkern verbunden sein können.

Beispiele für Monophenole der Formel (I) sind: Phenol, o-, m- und p-Kresol, o-, m- und p-Isopropylphenol, die entsprechenden Halogen- bzw. Alkoxyphenole, wie p-Chlorphenol bzw. p-Methoxyphenol, Salicylsäuremethylester, Salicylsäureethylester, ferner Monohydroxyverbindungen des Naphthalins, Anthracens und Phenanthrens, weiterhin 4-Hydroxypyridin und Hydroxychinoline. Vorzugsweise werden Phenol und gegebenenfalls substituierte Phenole, ganz besonders bevorzugt Phenol selbst eingesetzt.

Das erfindungsgemäße Verfahren kann sowohl mit Phosgen als auch mit Chlorkohlensäurearylestern durchgeführt werden. Für den Fall der Durchführung mit Phosgen entsteht zunächst der Chlorkohlensäurearylester der mit weiterem im Reaktionsgemisch vorhandenem Monophenol zum Diarylcarbonat umgesetzt wird.

Geht man von Chlorkohlensäurearylestern und einem Monophenol aus, können symmetrische oder unsymmetrische Diarylcarbonate erhalten werden.

Geeignete Chlorkohlensäurearylester für das erfindungsgemäße Verfahren sind solche der Formel (II)

Ar-OCOCl (II),

worin Ar die bei Formel (I) angegebene Bedeutung hat.

Geeignete Mischhydroxide im Sinne der Erfindung sind Verbindungen der allgemeinen Formel (III)

[M(II)₁₋ₓ M(III)ₓ M(IV)_{y} (OH)₂] Aⁿ⁻z/n m H₂O (III),

in welcher
- M (II): für ein divalentes Metallkation steht und
- M (III): für ein trivalentes Metallkation steht und
- M(IV): für ein tetravalentes Metallkation steht und
- x: für eine Zahl von 0,1 bis 0,5 steht und
- y: für eine Zahl von 0 bis 0,5 steht
- z: für 1 + y steht und
- m: für eine Zahl von 0 bis 32 steht
- A: für ein Anion wie CO₃²⁻, OH-, SO₄²⁻, NO₃⁻, CrO₄²⁻ oder Cl- steht, bevorzugt CO₃²⁻, OH-, SO₄²⁻,
- n: für 1 oder 2 steht

Als Beispiele für Metallkation M(II) seien genannt:
divalente Metallkationen wie Be, Mg, Ca, Zn, Fe, Mn, Co, Ni, Cu, Cd, bevorzugt sind Mg, Ni, Zn und Fe, besonders bevorzugt sind Mg, Ni und Zn.

Als Beispiele für Metallkation M(III) seien genannt:
trivalente Metallkationen wie Al, Ga, Ni, Co, Fe, Mn, Al, Cr, Fe, Sn, V, bevorzugt sind Al, Cr, Fe, besonders bevorzugt ist Al.

Als Beispiele für Metallkation M(IV) seien genannt: tetravalente Metallkationen wie Ti, Zr und Hf, bevorzugt sind Ti und Zr, besonders bevorzugt Ti.

In den Mischhydroxiden können auch mehrere verschiedene Metallkationen M(II) oder Metallkationen M(III) als auch Metallkationen M(II) oder Metallkationen M(III) desselben Elements in verschiedener Wertigkeit nebeneinander vorkommen.

Die erfindungsgemäß verwendeten Mischhydroxiden können eine Schichtstruktur aus Polykationen und -anionen, wie beispielsweise Hydrotalcit, oder eine davon abweichende Struktur wie beispielsweise Ettringit, besitzen.

Es kommen sowohl Mischhydroxide aus natürlichen Quellen, d.h. verschiedene Mineralien wie z.B.

Hydrotalcit Mg₆Al₂(OH)₁₆CO₃·4H₂O

Manasseit Mg₆Al₂(OH)₁₆CO₃·4H₂O

Pyroaurit Mg₆Fe₂(OH)₁₆CO₃·4.5H₂O

Sjörgrenit Mg₆Fe₂(OH)₁₆CO₃·4.5H₂O

Stichtit Mg₆Cr₂(OH)₁₆CO₃·4H₂O

Barbertonit Mg₆Cr₂(OH)₁₆CO₃·4H₂O

Takovit Ni₆Al₂(OH)₁₆CO₃·OH·4H₂O

Reevesit Ni₆Fe₂(OH)₁₆CO₃·4H₂O

Desautelsit Mg₆Mn₂(OH)₁₆CO₃·4H₂O

Hydrocalumit [Ca₂Al(OH)₆]OH·6H₂O

Magaldrat[Mg₁₀Al₅(OH)₃₁](SO₄)₂·mH₂O

Ettringit [Ca₆Al₂(OH)₁₂](SO₄)₃·26H₂O

aber auch synthetische, im Allgemeinen durch Fällung aus Lösungen von Vorprodukten, beispielsweise Metallsalzen oder Metalloxiden, und Basen hergestellt, in Frage.

Solche Mischhydroxide und deren Herkunft bzw. Herstellverfahren für solche Verbindungen sind beispielsweise in Clays and Clay Minerals 25 (1977) 14, 23(1975) 369, Catalysis Today 11 (1991) 173, Chimia 24 (1970) 99, und EP-A 749 941, EP-A 421 677, EP-A 684 872, EP-A 0749941, DE-OS 2 024 281 und WO 95/17246 beschrieben.

Besonders geeignet als heterogene Katalysatoren sind Mischhydroxide mit Hydrotalcitstruktur, beispielsweise Mischhydroxide von Magnesium, Zink, Nickel, Aluminium, Kobalt, Zinn und Titan.

Die Mischhydroxide im Sinne der Erfindung können in kristalliner Form in verschiedenen Modifikationen vorliegen. Sie können ganz oder teilweise amorph sein und getrocknet, teilgetrocknet oder als Hydrate eingesetzt werden.

Durch Reaktion von gemischten Metallsalzen in Gegenwart von Basen bei Temperaturen von 80 bis 100°C entstehen zunächst Hydroxycarbonate, die bei höheren Calcinierungstemperaturen unter Decarboxylierung und mit fortschreitender Entwässerung in die wasserfreien Mischhydroxide übergehen. So geht bei Calcinierung über 500°C Hydrotalcit Mg₆Al₂(OH)₁₆CO₃·4H₂O in Mg₆Al₂O₅(OH)₂ über. Je nach Art des Ausgangshydroxids bzw. -hydroxidcarbonats können bei der Calcinierung verschiedene der obengenannten Modifikationen des Mischhydroxids durchlaufen werden.

Bevorzugte Mischhydroxide besitzen BET-Oberflächen von 0,1 bis 500 m²/g, besonders bevorzugt solche von 0,5 bis 450 m²/g und ganz besonders bevorzugte solche von 1 bis 300 m²/g.

Die Katalysatoren können z.B. als Pulver oder Formkörper eingesetzt werden und nach der Umsetzung z.B. durch Filtration, Sedimentation oder Zentrifugieren abgetrennt werden. Für den Fall der Anordnung als Festbett werden die Metallate vorzugsweise als Formkörper, z.B. als Kugeln, Zylinder, Stäbchen, Hohlzylinder, Ringe usw. eingesetzt.

Die Mischhydroxid-Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monophenol, verwendet.

Im Falle einer kontinuierlichen Fahrweise im Gegen- oder Gleichstrom oder in der Rieselphase oder in der Gasphase am Festbettkatalysator werden Katalysatorbelastungen von 0,1 bis 20 g Monophenol pro g Katalysator pro Stunde, bevorzugt 0,2 bis 10 g · g⁻¹ · h⁻¹ und besonders bevorzugt von 0,2 bis 5 g · g⁻¹ · h⁻¹ verwendet.

Die in diskontinuierlichen Versuchen verwendeten Mischhydroxide können bei gleichen Einsatzstoffen ohne Reinigung wiederholt eingesetzt werden. Bei einem Wechsel der Einsatzstoffe werden die Mischhydroxide zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzten Mischhydroxide über lange Zeit im Reaktor verbleiben. Eine Regenerierung kann gegebenenfalls z.B. durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 20 Gew.-% Sauerstoff enthaltenden Verdünnungsgasen wie Stickstoff, oder Kohlendioxid oder durch Kohlendioxid allein bei 200 bis 800°C erfolgen. Die bevorzugte Regenerierungstemperatur liegt bei 150 bis 700°C, besonders bevorzugt bei 200 bis 600°C.

Das erfindungsgemäße Verfahren wird bei einer Temperatur im Bereich von 50 bis 450°C, bevorzugt 100 bis 400°C, besonders bevorzugt 100 bis 350°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck von 0,05 bis 20 bar, vorzugsweise 1 bis 5 bar, durchgeführt.

Man kann das erfindungsgemäße Verfahren gegebenenfalls unter Verwendung von Lösungsmitteln wie aliphatischen und aromatischen Kohlenwasserstoffen, eg. Hexan, Octan, Benzol, isomeren Xylolen, Diethylbenzol, Alkylnaphthalinen, Biphenyl oder halogenierten Kohlen-wasserstoffen, wie Dichlormethan und Trichlorethylen durchführen.

Man kann das erfindungsgemäße Verfahren sowohl in der Gasphase als auch in der Flüssigphase durchführen.

Vorzugsweise wird das Verfahren in der Schmelze durchgeführt, beispielsweise, indem man in eine Suspension eines Mischhydroxids in einer Schmelze des Monophenols der Formel (I) Phosgen oder einen Chlorkohlensäurearylester der Formel (II) einleitet und nach Beendigung der Reaktion den Katalysator z.B. durch Filtration oder Zentrifugieren abtrennt.

Das Verfahren wird in der Gasphase durchgeführt, indem man Phosgen und Monophenol verdampft und das Gemisch über ein Bett eines in einem Rohr angeordneten stückigen Katalysators leitet.

Eine weitere bevorzugte Ausführungsform der Synthese ist die Begasung einer Schmelze des Monophenols der Formel (I) mit darin suspendiertem MischhydroxidKatalysator mit Phosgen oder Phosgen-Chlorwasserstoff-Gemischen oder mit Chlorkohlensäurearylestern der Formel (II) in einer kontinuierlich arbeitenden Blasensäule bzw. Blasensäulen-Kaskade.

Eine weitere bevorzugte Durchführungsform ist das Gleichstromverfahren, bei dem Monophenole der Formel (I) und Phosgen bzw. Chlorkohlensäurearylester der Formel (II) im Gleichstrom beispielsweise von oben her auf eine in einem Rohr angeordnete Katalysatorschüttung aufgebracht und unten am Fuß des Rohres Chlorwasserstoff und Phosgenierungsprodukte abgezogen werden.

Eine weitere bevorzugte Ausführungsform ist die Durchführung der erfindungsgemäßen Umsetzung im Gegenstrom in der Rieselphase, wobei das Monophenol der Formel (I) als Schmelze oder in Form einer Lösung oben auf ein Bett von Mischhydroxid gegeben wird und diesem Flüssigkeitstrom von unten ein Strom von Phosgen oder Chlorkohlensäurearylester entgegengeschickt wird. Zweckmäßig wird diese Ausführungsform in einem senkrecht stehenden Rohrreaktor durchgeführt, die auch Zwischenböden zur verbesserten Verteilung von Gas- und Flüssigkeitsstrom enthalten kann.

Eine weitere bevorzugte Ausführungsform ist das Gasphasenverfahren bei Temperaturen von 150 bis 450°C, bevorzugt 200 bis 350°C mit Drucken von 0,05 bis 20, bevorzugt 0,1 bis 4 bar, besonders bevorzugt 0,1 bis 3 bar.

Bei diesem Verfahren wird der Druck so mit der Temperatur variiert, dass die Komponenten in der Gasphase bleiben und nicht auf der Katalysatorschüttung kondensieren.

Das molare Verhältnis der Reaktionspartner Monophenole der Formel (I) zu Phosgen beträgt 0,5 bis 8:1, bevorzugt 1,5 bis 3:1. Das äquivalente molare Verhältnis ist in diesem Fall 2:1.

In entsprechender Weise wird das Monophenol mit einem Chlorkohlensäurearylester im Molverhältnis von 0,25 bis 4:1, bevorzugt 0,8 bis 1,5:1 umgesetzt. In diesem Fall beträgt das molare Verhältnis 1:1.

Das erfindungsgemäß durch heterogene Katalyse gewonnene rohe Diarylcarbonat ist häufig schon sehr rein und kann nach Entgasung von restlichem Chlorwasserstoff oder anderen flüchtigen Stoffen bereits in dieser Form für viele Zwecke verwendet werden. Für anspruchsvollere Anwendungen kann das Diarylcarbonat gegebenenfalls z.B. durch Destillation oder Kristallisation nach bekannten Verfahren weiter gereinigt werden.

Weiterer Gegenstand der Erfindung ist ein Verfahren zur Herstellung von Diarylcarbonaten unter Verwendung von geträgerten Katalysatoren. Als heterogene Katalysatoren eignen sich in diesem Fall insbesondere Verbindungen der Formel (III)

[M(II)₁₋ₓ M(III)ₓ M(IV)_{y} (OH)₂] Aⁿ⁻_{z/n} · m H₂O (III)

auf Trägermaterialen, die auch dotiert sein können.

Die Verbindungen der Formel (III) können auch mit weiteren Substanzen als Bestandteil einer Katalysatorformulierung vermengt werden um gegebenenfalls synergistische Effekte zu generieren. Hierfür eignet sich beispielsweise Siliciumdioxid, Graphit, Titandioxid mit Rutil- oder Anatas-Struktur, Zirkondioxid, Aluminiumoxid, Siliziumcarbide oder deren Gemische, bevorzugt Titandioxid, Zirkondioxid, Aluminiumoxid oder deren Gemische.

Die Reaktion zum Diarylcarbonat kann mehrstufig durchgeführt werden. Diese kann diskontinuierlich, bevorzugt kontinuierlich als Fließ- oder Festbettverfahren, bevorzugt als Festbettverfahren, besonders bevorzugt in Rohrbündelreaktoren an den heterogenen Katalysatoren durchgeführt werden.

Eine bevorzugte Ausführungsform besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen.

Die Reaktionswärme der Umsetzung kann in vorteilhafter Weise zur Erzeugung von Hochdruck-Wasserdampf genutzt werden.

### Beispiele

Die eingesetzten Katalysatoren sind käufliche Produkte oder wurden nach bekannten Verfahren hergestellt (s. Catalysis Today 11 (1991) 173, EP-A 421 677, EP-A 749 941, WO 95/17248, EP-A 684 872, DE-OS 2 024 282).

### Beispiel 1

In einem Planschlifftopf mit Strombrechern, einem Begasungsrührer und Rückflußkühler wurden 141 g (1,50 mol) Phenol in Gegenwart von 14,1 g (10 Gew.-% bezogen auf Phenol) eines pulverförmigen Hydrotalcits (Molverhältnis Mg/Al = 2: 1) bei 140°C mit 0,75 mol/h Phosgen kontinuierlich begast. Nach etwa 2 h Reaktionszeit betrug der Phenolumsatz 29,8 %, wobei nur Diphenylcarbonat (57,6 g) gebildet wurde. Die Selektivität zum Carbonat betrug >99,7%.

### Beispiel 2

Das Beispiel 1 wurde mit 14,1 g eines pulverförmigem Zink-Aluminium-Hydroxids (Molverhältnis Zn/Al= 2:1) bei 140°C wiederholt. Nach 2 h Reaktionszeit betrug der Phenolumsatz 15,2%, wobei 0,03 g Chlorameisensäurephenylester und 23,9 g Diphenylcarbonat gebildet wurde. Die Selektivität zum Carbonat betrug ca. 90%.

### Beispiel 3

Das Beispiel 1 wurde mit 14,1 g eines pulverförmigen Nickel(II)-Aluminium- Hydroxids (Molverhältnis Ni/Al = 2:1) bei 140°C wiederholt. Nach 2 h Reaktionszeit betrug der Phenolumsatz 11,2 % wobei 0,6 g Chlorameisensäurephenylester und 17,4 g Diphenylcarbonat gebildet wurde. Die Selektivität zum Carbonat betrug ca. 99%.

### Beispiel 4

Das Beispiel 1 wurde mit 14,1 g eines pulverförmigen Hydrotalcits (Molverhältnis Mg/Al 7:3) der Firma Condea bei 140°C wiederholt. Nach 2 h Reaktionszeit betrug der Phenolumsatz 24,4 % wobei 39,0 g Diphenylcarbonat gebildet wurden. Die Carbonatselektivität betrug >99 %.

### Beispiel 5

Das Beispiel 1 wurde mit 14,1 g eines pulverförmigen Magnesium-Zinn(II)-Hydroxids (Molverhältnis Mg/Sn = 1,0/0,034) bei 140°C wiederholt. Nach 2 h Reaktionszeit betrug der Phenolumsatz 24,6 %, wobei 39,2 g Diphenylcarbonat gebildet wurden. Die Selektivität zum Carbonat war größer als 99 %.

### Beispiel 6

Das Beispiel 1 wurde mit 14,1 g eines pulverförmigen Magnesium-Titan(IV)-Hydroxids (Molverhältnis Mg/Ti = 1,0/0,050) bei 140°C wiederholt. Nach 2 h Reaktionszeit betrug der Phenolumsatz 26,6 %, wobei 42,4 g Diphenylcarbonat gebildet wurden. Die Carbonatselektivität war >99 %.

### Beispiel 7

Das Beispiel 1 wurde mit 14,1 g eines pulverförmigen Hydrotalcits (Molverhältnis = Mg/Al 7:3) der Firma Condea bei 140°C wiederholt. Nach 2 h Reaktionszeit betrug der Phenolumsatz 24,4 %, wobei 39,0 g Diphenylcarbonat gebildet wurden. Die Carbonatselektivität war >99 %.

### Beispiel 8

Das Beispiel 1 wurde mit 14,1 g eines pulverförmigen Nickel(II)-Magnesium-Aluminium-Hydroxids (Molverhältnis Ni/Mg/Al = 0,14/2,34/1,0) bei 140°C wiederholt. Nach 2 h Reaktionszeit betrug der Phenolumsatz 19,9 %, wobei 31,0 g Diphenylcarbonat gebildet wurden. Die Selektivität zum Carbonat betrug ca. 97 %.

### Beispiel 9

Das Beispiel 1 wurde mit 1,41 g eines pulverförmigen Titan(IV)-Magnesium-Aluminium-Hydroxids (Molverhältnis Ti/Mg/Al = 0,26/2,63/1,0) bei 140°C wiederholt. Nach 2 h Reaktionszeit betrug der Phenolumsatz 22,8 %, wobei 36,5 g Diphenylcarbonat gebildet wurden. Die Selektivität zum Carbonat betrug >99 %.

### Beispiel 10

In einem Dreihalskolben mit Thermometer und Rückflußkühler wurde ein Gemisch aus 9,4 g (0,10 mol) Phenol und 15,7 g (0,10 mol) Chlorameisensäurephenylester in Gegenwart von 0.94 g (10 Gew.-% bezogen auf Phenol) eines pulverförmigen Hydrotalcits (Molverhältnis = Mg/Al 2:1) auf 140°C erhitzt. Nach 5 h Reaktionszeit waren 90,7 % des Phenols zu Diphenylcarbonat umgesetzt.

### Beispiel 11

Das Beispiel 10 wurde mit 0,94 g eines pulverförmigen Zink-Aluminium-Hydroxids (Molverhältnis = 2:1) bei 140°C wiederholt. Nach 1 h Reaktionszeit betrug der Phenolumsatz zu Diphenylcarbonat 99,8 %. Die Carbonatselektivität war >99 %.

### Beispiel 12

Das Beispiel 10 wurde mit 0,94 g eines Nickel(II)-Aluminium-Hydroxids (Molver-hältnis = 2:1) bei 140°C wiederholt. Nach 3 h Reaktionszeit betrug der Phenolumsatz zu Diphenylcarbonat 97,5 %. Die Carbonatselektivität war >99 %.

### Beispiel 13

Das Beispiel 10 wurde mit 0,94 g eines pulverförmigen Magnesium-Zinn-Hydroxids (Molverhältnis = 1,0/0,034) bei 140°C wiederholt. Nach 2 h Reaktionszeit betrug der Phenolumsatz zu Diphenylcarbonat 49,7 %. Die Selektivität zum Carbonat war >99 %.

### Beispiel 14

Das Beispiel 10 wurde mit 0,94 g eines pulverförmigen Magnesium-Titan(IV)-Hydroxids (Molverhältnis = 1,0/0,050) bei 140°C wiederholt. Nach 2 h Reaktionszeit betrug der Phenolumsatz zu Diphenylcarbonat 86,1 %. Die Carbonatselektivität war >99 %.

### Beispiel 15

Das Beispiel 10 wurde mit 0,94 g eines pulverförmigen Hydrotalcits (Molverhältnis = Mg/Al 7:3) der Forma Condea bei 140°C wiederholt. Nach 1 h Reaktionszeit betrug der Phenolumsatz zu Diphenylcarbonat 98,8 %. Die Selektivität zum Carbonat war >99 %.

### Beispiel 16

Das Beispiel 10 wurde mit 0,94 g eines pulverförmigen Titan(IV)-Magnesium-Aluminium-Hydroxids (Molverhältnis = 0,26/2,63/1,0) wiederholt. Nach 1 h Reaktionszeit betrug der Phenolumsatz zu Diphenylcarbonat 98,6 %. Die Selektivität zum Carbonat war >99 %.

### Vergleichsbeispiel 1

Beispiel 1 wurde ohne Zusatz von Mischhydroxid bei 140°C wiederholt. Nach 2 h Reaktionszeit betrug der Phenolumsatz weniger als 0,2 %.

### Vergleichsbeispiel 2

Beispiel 1 wurde in Gegenwart von pulverförmigen Aluminiumoxid 507-C-I bei 140°C wiederholt. Nach 2 h Reaktionszeit betrug der Phenolumsatz 41% und die Carbonatselektivität war > 99,5%.

### Vergeichsbeispiel 3

Beispiel 11 wurde in Gegenwart von pulverförmigen Aluminiumoxid 507-C-I bei 140°C wiederholt. Nach 2 h Reaktionszeit betrug der Phenolumsatz 90% und die Selektivität zum Carbonat war > 99%.

## Patentansprüche

1. Verfahren zur Herstellung von Diarylcarbonaten durch Umsetzung von Monophenolen mit Phosgen oder Chlorkohlensäurearylestern, **dadurch gekennzeichnet, dass** die Umsetzung in Gegenwart von einer oder mehreren Verbindungen der allgemeinen Formel (III)
[M(II)₁₋ₓ M(III)ₓ M(IV)_{y} (OH)₂] Aⁿ⁻_{z/n} · m H₂O (III)
in welcher
M (II) für ein divalentes Metallkation steht und
M (III) für ein trivalentes Metallkation steht und
M(IV) für ein tetravalentes Metallkation steht und
x für eine Zahl von 0,1 bis 0,5 steht und
y für eine Zahl von 0 bis 0,5 steht
z für 1 + y steht und
m für eine Zahl von 0 bis 32 steht
A für ein Anion wie CO₃²⁻, OH-, SO₄²⁻, NO₃⁻, CrO₄²⁻ oder Cl⁻ steht,
n für 1 oder 2 steht
als heterogene Katalysatoren durchgeführt wird.

2. Verfahren nach Anspruch 1, **dadurch gekennzeichnet, dass** die Umsetzung bei einer Temperatur von 50 bis 450°C, bei einem Druck von 0,05 bis 20 bar durchgeführt wird.

3. Verfahren nach Anspruch 1 oder 2, **dadurch gekennzeichnet, dass** die Katalysatoren nach der BET-Methode bestimmte Oberflächen von 0,1 bis 400 m²/g aufweisen und in Mengen von 0,5 bis 100 Gew.-%, bezogen auf die Menge an Monophenol, bei nicht vollkontinuierlicher Fahrweise bzw. mit Belastungen von 0,1 bis 20 g Monophenol pro g Katalysator pro Stunde bei vollkontinuierlicher Fahrweise, eingesetzt werden.

4. Verfahren nach Anspruch 1, **dadurch gekennzeichnet, dass** als divalente Metallkationen M(II) Mg, Ni, Zn, als trivalente Metallkationen M(III) Al und als tetravalente Metallkationen M(IV) Ti oder Zr eingesetzt werden.

5. Verfahren nach Anspruch 1, **dadurch gekennzeichnet, dass** die Herstellung von Diarylcarbonaten kontinuierlich erfolgt.

6. Verfahren nach Anspruch 1, **dadurch gekennzeichnet, dass** das Verfahren bei einer Temperatur von 100 bis 350°C und einem Druck von 0,05 bis 20 bar betrieben wird.

7. Verfahren nach Anspruch 1, **dadurch gekennzeichnet, dass** die Reaktion in der Gasphase erfolgt.

8. Verfahren nach Anspruch 1, **dadurch gekennzeichnet, dass** die Umsetzung im Gegenstrom in der Rieselphase durchgeführt wird.

9. Verfahren nach Anspruch 1, **dadurch gekennzeichnet, dass** der Katalysator aus einer geträgerten Aktivphase der Formel (III) besteht.

10. Diarylcarbonat erhältlich mit dem Verfahren gemäß einem der Ansprüche 1 bis 9.

11. Verwendung eines Katalysators gemäß einem der Ansprüche 1 bis 10 zur Herstellung von Diarylcarbonaten.
